(19) Europäisches Patentamt
European Patent Office
Office européen des brevets

(11) Veröffentlichungsnummer: **0 013 958**
**B1**

(12) # EUROPÄISCHE PATENTSCHRIFT

(45) Veröffentlichungstag der Patentschrift: **10.02.82**

(51) Int. Cl.³: **C 07 C 68/00,** C 08 G 63/62

(21) Anmeldenummer: **80100267.6**

(22) Anmeldetag: **21.01.80**

(54) **Verfahren zur Herstellung von Kohlensäureestern und Polycarbonaten.**

(30) Priorität: **30.01.79 DE 2903507**

(43) Veröffentlichungstag der Anmeldung: **06.08.80 Patentblatt 80/16**

(45) Bekanntmachung des Hinweises auf die Patenterteilung: **10.02.82 Patentblatt 82/6**

(84) Benannte Vertragsstaaten: **DE FR GB IT NL**

(56) Entgegenhaltungen:
**US-A-2 834 799**
**Journal of Organic Chemistry, Vol. 25 (1960) p. 1874–1876**

(73) Patentinhaber: **BAYER AG, Zentralbereich Patente, Marken und Lizenzen, D-5090 Leverkusen 1, Bayerwerk (DE)**

(72) Erfinder: **Heitz, Walter, Prof. Dr., Am Schmidtborn 5, D-3575 Kirchhain 1 (DE)**
Erfinder: **Ball, Peter, Dr., Am Burger Wald 71, D-8267 Neu Oetting (DE)**

**EP 0 013 958 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von Kohlensäureestern und Polycarbonaten

Gegenstand der vorliegenden Erfindung ist ein Verfahren zur Herstellung von Kohlensäureestern und von Polycarbonaten, wie es in den Patentansprüchen 1 und 2 angegeben ist.

US-PS 2 834 799 beschreibt unter anderem die Umsetzung von Urethanen mit Alkohlen und $BF_3$. Hierbei wirkt jedoch $BF_3$ als Reaktionspartner und nicht als Katalysator.

Journal of Organic Chemistry, Vol. 25, (1960), Seiten 1874–1876 beschreibt die Reaktion von Urethanen mit Alkoholen unter verschiedenen katalytischen Bedingungen. So estert beispielsweise Ethyl-N-ethyl-N-phenylcarbamat mit Benzylalkohol und mit Isobutylalkohol unter Basenkatalyse um; lediglich Ethyl-N,N-diphenylcarbamat setzt sich mit Isobutylalkohol zum Diisobutylcarbonat unter Basenkatalyse um. Dagegen reagiert Ethylcarbamat mit Isobutylalkohol unter Basenkatalyse nicht. Demzufolge war es nicht zu erwarten, dass die N,N-unsubstituierten Urethane gemäss vorliegender Erfindung mit Mono-alkoholen oder mit Polyalkoholen zu Carbonaten reagieren, zumal die Rückspaltung der unsubstituierten Urethane zu befürchten war.

Aus der «Zeitschrift für Naturforschung, Band 1, 1946, Seiten 518 ff» ist die Umsetzung von Harnstoffen mit Alkoholen zu Urethanen beschrieben.

Eine Weiterreaktion der Urethane zu Estern ist dort nicht beschrieben. Mit dem in dieser Literaturstelle angegebenen Katalysatoren lassen sich bei Verlängerung der Reaktionszeit geringe Mengen Carbonat durch Gaschromatographie nachweisen. (Siehe «Angewandte Chemie», 92 Jahrgang 1980, Heft 9, Seiten 742–743, nachveröffentlicht). Somit wird auch durch die Literaturstelle «Zeitschrift für Naturforschung, Band 1, 1946, Seiten 518 ff,» der Gegenstand der vorliegenden Erfindung nicht nahegelegt.

Als Monoalkohole bzw. Polyalkohole kommen vorzugsweise primäre Alkohole in Betracht, die eine beliebige Anzahl von C-Atomen enthalten können. Geeignete Alkohole sind aliphatische, cycloaliphatische, araliphatische und heterocyclische Alkohole. Polyalkohole im Sinne vorliegender Erfindung sind beispielsweise solche mit 2 bis 3 alkoholischen OH-Gruppen, wobei bei entsprechendem Abstand der alkoholischen OH-Gruppen im Molekül cyclische Kohlensäureester entstehen können.

Die geeigneten Monoalkohole haben vorzugsweise 1 bis 20 C-Atome.

Beispiele für Monoalkohole sind Methanol, Äthanol, Propanole, Butanole, Pentanole, Hexanole, Octanole, Stearylalkohol, Methylolcyclohexan, Benzylalkohol, 2-Phenyläthanol, 2-Naphthyläthanol, Furfurylalkohol etc.

Geeignete Polyalkohole haben vorzugsweise 5 bis 20 C-Atomen.

Beispiele für Polyalkohole sind Hexan-1,6-diol, Decandiole, 1,4-Dimethylolcyclohexan, 1,4-Dimethylolbenzol, 3(4), 8(9) Bis(hydroxymethyl)-tricyclo-[5,2,1,$0^{2,6}$]decan

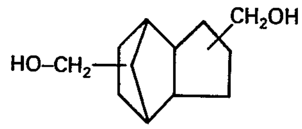

und Bis-hydroxymethyl-tetrahydrofurane etc.

Erfindungsgemäss geeignete Urethane sind solche der Formel Ia und Ib

$$R_1-O-\overset{\displaystyle O}{\overset{\|}{C}}-NH_2 \qquad (Ia)$$

$$H_2N-\overset{\displaystyle O}{\overset{\|}{C}}-O-R_4-O-\overset{\displaystyle O}{\overset{\|}{C}}-NH_2 \qquad (Ib)$$

worin

$R_1$ ein primäres $C_1$–$C_{28}$-Alkyl ist

$R_4$ ein zweiwertiger Rest ist, der sich aus einem diprimären Dialkohol mit vorzugsweise 2 bis 20 C-Atomen durch Entfernen der beiden Hydroxygruppen ableitet.

Erfindungsgemäss geeignete Urethane der Formel Ia sind beispielsweise Isooctylurethan (Carbaminsäureisooctylester), Hexylurethan oder Butylurethan (Carbaminsäurebutylester).

Erfindungsgemäss geeignete Urethane der Formel Ib sind beispielsweise 1,6-Hexandiol-bis-urethan oder 1,10-Decandiol-bisurethan.

Erfindungsgemässe geeignete Katalysatoren sind beispielsweise Verbindungen der 1. bis 6. Haupt- und Nebengruppen sowie der 7. und 8. Nebengruppen des Periodischen Systems der Elemente. (Siehe Hollemann-Wiberg, Lehrbuch der Anorganischen Chemie, 37–39, Auflage, Verlage Walter De Gruyter u. Co., Berlin 1956).

Erfindungsgemäss geeignete Verbindungen der 1. Haupt- und 1. Nebengruppe sind sowohl salzartige als auch covalente Verbindungen der Metalle dieser Gruppen des periodischen Systems wie LiBr, Butyllithium, LiCl, LiJ, $NaOCH_3$, CuCl, $AgOCO-CH_3$ etc.

Erfindungsgemäss geeignete Verbindungen der 2. Haupt- und 2. Nebengruppe sind sowohl salzartige als auch covalente Verbindungen der Metalle dieser Gruppen des periodischen Systems wie $MgCl_2$, $Be(O-CO-CH_3)_2$, Grignard-Verbindungen, $ZnCl_2$, $ZnSO_4$, $Mg(OC_2H_5)_2$ etc.

Erfindungsgemäss geeignete Verbindungen der 3. Haupt- und 3. Nebengruppe sind insbesondere covalente Verbindungen der Elemente dieser Gruppen des periodischen Systems wie $B-(-O-C_6H_5)_3$, $B\,(C_6H_5)_3$, $Al(OR)_3$, (R = aliphatischer oder aromatischer Kohlenwasserstoffrest) etc.

Erfindungsgemäss geeignete Verbindungen der 4. Haupt- und 4. Nebengruppe sind insbesondere covalente Verbindungen der Elemente dieser Gruppen des periodischen Systems wie Orthotitanate (z.B. Tetrabutyl-orthotitanat), Orthostannate (z.B. Tetraphenyl-orthostannat) etc.

Erfindungsgemäss geeignete Verbindungen der 5. Haupt- 5. Nebengruppe sind insbesondere covalente Verbindungen der Elemente dieser Gruppen des periodischen Systems wie Amine (z.B. Diazabicyclooctan), Phosphine (z.B. Triphenylphosphin, Tri-n-octylphosphin), Phosphinoxide (z.B. Triphenylphosphinoxid), Phosphonsäureester (z.B. Tributylphosphit, Triphenylphosphit) etc.

Erfindungsgemäss geeignete Verbindungen der 6. Haupt- und 6. Nebengruppe sind sowohl covalente als auch salzartige Verbindungen der Elemente dieser Gruppen des periodischen Systems wie Thioäther (z.B. Diphenylsulfid) Thiolate (z.B. Na-thiophenolat) etc.

Erfindungsgemäss geeignete Verbindungen der 7. Nebengruppe sind sowohl covalente als auch salzartige Verbindungen der Elemente dieser Gruppe des periodischen Systems (z.B. Mangan (II)acetat).

Erfindungsgemäss geeignete Verbindungen der 8. Nebengruppe sind sowohl covalente als auch salzartige Verbindungen der Elemente dieser Gruppe des periodischen Systems (z.B. Eisen (III)acetylacetonat).

Besonders bevorzugte Katalysatoren für das erfindungsgemässe Verfahren sind Kombinationen der vorstehend genannten Verbindungen, wobei Elektronendonator- und Elektronenakzeptor-Eigenschaften der Katalysatorkombination aufeinander abzustimmen sind, d.h. Kombinationen von Elektronendonatoren und Elektronenakzeptoren einzusetzen sind.

Besonders wirksam sind Kombinationen von Lewissäuren und -basen, insbesondere von Verbindungen der 1. bis 3. Hauptgruppe mit Verbindungen der 4. bis 6. Hauptgruppe des periodischen Systems der Elemente.

Beispiele derartiger Kombinationen sind Aluminiumalkoholate in Kombination mit Aminen, Phosphinen oder Phosphinoxiden, oder Magnesiumalkoholate in Kombination mit Thioethern.

Die erfindungsgemäss geeigneten Katalysatoren werden in Mengen von $10^{-3}$ bis 5 Mol-%, bezogen auf Mole an jeweils eingesetzten Urethanen eingesetzt, und zwar gilt dies sowohl für den Einsatz von einzelnen Katalysatoren als auch von Katalysatorkombinationen. Das molare Verhältnis der Katalysatorarten in den Katalysatorkombinationen kann zwischen 1:10 und 10:1 variieren.

Zur Durchführung der erfindungsgemässen Reaktion werden die Komponenten, d.h. Urethan, Alkohol und Katalysator zusammen unter Rühren auf Temperaturen von mindestens 120 °C erwärmt, und 5 bis 10 Stunden langsam auf 270 °C erwärmt und ausreagieren lassen. Anschliessend wird der Ansatz bei niedrigerer Temperatur im Vakuum schonend destillativ getrennt und der Kohlensäureester isoliert, wobei die Ausbeuten sehr gut sind.

Da im allgemeinen die Alkoholkomponente im Überschuss angewandt wird, variiert das Molverhältnis von Urethangruppe zu Alkohol zwischen 1:1 und etwa 1:5, vorzugsweise zwischen 1:1 und 1:2.

Die nach dem erfindungsgemässen Verfahren hergestellten Kohlensäureester sind bekannte Verbindungen und eignen sich bekanntlich als Lösungsmittel in der Organischen Chemie sowie als Zwischen- bzw. Ausgangsprodukt für die verschiedensten chemischen Reaktionen.

Insbesondere eignen sie sich in bekannter Weise (Vgl. DT-PS 1031512, Beispiel 1) zur Synthese von Polycarbonaten durch Umsetzung mit Diolen.

Die Herstellung von Oligo- und Polycarbonaten lässt sich einstufig nach dem erfindungsgemässen Verfahren aus den genannten Urethanen, den genannten Monoalkoholen und primären Dialkoholen erreichen, wobei als Dialkohole aliphatische, cycloaliphatische und araliphatische geeignet sind unter dem Vorbehalt, dass die Bildung cyclischer Carbonate unter den Verfahrensbedingungen praktisch nicht auftritt.

Vorzugsweise ist hierbei das Molverhältnis von Urethan zu Dialkoholen etwa 1:1; die Molmenge aus Monoalkoholen bezogen auf Molmenge an Dialkoholen, richtet sich nach der jeweils angestrebten Kettenlänge, wobei man bereits vorliegende Estergruppen von Monoalkoholen (resultierend beispielsweise aus Verbindungen der Formel Ia) mitzuberücksichtigen sind, und kann, da im Überschuss eingesetzt, destillativ entfernt werden. Durch die Wahl der Reaktionstemperatur und der Reaktionszeit wird das Molekulargewicht der Oligo- bzw. Polycarbonate ausserdem in bekannter Weise reguliert. Nach dem erfindungsgemässen Verfahren sind somit auf einfache Weise Oligo- und Polycarbonate von primären Dialkoholen mit verschiedenen Molekulargewichten herstellbar.

Für die, für die erfindungsgemässe Oligo- und Polycarbonatherstellung geeigneten Monourethane oder Bisurethane und Katalysatoren sind die eingangs gemachten Definitionen zur Erläuterung der erfindungsgemässen Herstellung von Kohlensäureestern ebenfalls zutreffend.

Für die erfindungsgemässe Oligo- und Polycarbonatherstellung sind je nach Ansatz und Temperaturführung zwischen 2 und 10 Stunden erforderlich. Das jeweils gewünschte Ende der Reaktion kann beispielsweise viskosimetrisch ermittelt werden.

Als primäre Dialkohole sind vorzugsweise solche mit 5 bis 20 C-Atomen, geeignet; Beispiele sind die bereits eingangs im Zusammenhang mit der erfindungsgemässen Herstellung von Kohlensäureestern genannten, wie Hexan-1,6-diol oder 1,4-Dimethylol-cyclohexan.

Die übrigen Reaktionsbedingungen der Oligo bzw. Polycarbonatherstellung, also Einsatz und Menge der einzusetzenden Katalysatoren sowie die Temperaturführung entsprechen denen der bereits beschriebenen Herstellung der monomeren Kohlensäureester.

Die nach dem erfindungsgemässen Verfahren erhaltenen Oligo- und Polycarbonate sind im Prinzip bekannt und geeignet, in bekannter Weise zur Herstellung von Formkörpern und Folien sowie als Beschichtungen und Additive für andere Kunststoffe. Die erfindungsgemäss erhältlichen

Oligo- und Polycarbonate sind somit geeignet für die bekannten Einsatzgebiete der thermoplastischen Polycarbonate, also etwa bezüglich der Oligocarbonate als sekundäre Weichmacher für beispielsweise PVC sowie etwa zur Modifizierung von hochmolekularen thermoplastischen Polycarbonaten beispielsweise gemäss US-PS 3 166 606.

Beispiel

Darstellung eines Kohlensäureesters

30,94 g Carbaminsäureisooctylester, 48,90 g Isooctanol, 0,40 g Triphenylphosphin und 2,0 ml einer 20%igen Lösung von Diisobutylaluminiumhydrid in Toluol wurden zusammen unter Rühren erwärmt. Bei ca. 165 °C beginnt die Mischung zu sieden. Die Mischung wurde nun so erwärmt, dass in einer aufgesetzten Vigrenx-Kolonne (Mantellänge 30 cm) ständiger Rückfluss von Isooctanol bestand. Während 7 h wurde die Temperatur kontinuierlich auf 270 °C erhöht; es waren dann 2,78 g (91,3% der Theorie) $NH_3$ abgeschieden und 24,95 g Flüssigkeit und etwas Feststoff abdestilliert (Isooctanol, Urethan, Harnstoff). Anschliessend wurde die Mischung fraktioniert destilliert; es resultierten eine Fraktion von 4,35 g mit $Kp_{14}$ 81,5 (Isooctanol) und eine Fraktion von 46,96 g mit $Kp_{1\sim 0,05}$ 98–102 Diisooctylcarbonat, Ausbeute 92,33% bezogen auf eingesetztes Urethan.

**Patentansprüche**

1. Verfahren zur Herstellung von Kohlensäureestern von Mono- oder Polyalkoholen durch Umsetzung von Urethanen der Formeln Ia oder Ib

$$R_1-O-\overset{\overset{O}{\|}}{C}-NH_2 \qquad (Ia)$$

$$H_2N-\overset{\overset{O}{\|}}{C}-O-R_4-O-\overset{\overset{O}{\|}}{C}-NH_2 \qquad (Ib)$$

worin

$R_1$ ein primäres $C_1$–$C_{18}$-Alkyl ist, und

$R_4$ ein zweiwertiger Rest, der sich aus einem diprimären Dialkohol durch Entfernen der beiden Hydroxygruppen ableitet,

mit Mono- oder mit Polyalkoholen im Molverhältnis Alkohol zu Urethangruppe von mindestens 1 zu 1, dadurch gekennzeichnet, dass die Umsetzung in Gegenwart von $10^{-3}$ bis 5 Mol-%, bezogen auf Mole an jeweils eingesetzten Urethanen, Katalysatoren erfolgt, wobei man unter Rühren auf Temperaturen von mindestens 120 °C erwärmt, während 5 bis 10 Stunden langsam auf 270 °C erwärmt und ausreagieren lässt.

2. Verfahren zur Herstellung von Oligo- und von Polycarbonaten durch Umsetzung von Monourethanen der Formel (Ia)

$$R_1-O-\underset{\underset{O}{\|}}{C}-NH_2 \qquad (Ia)$$

oder Bisurethanen der Formel (Ib)

$$H_2N-\overset{\overset{O}{\|}}{C}-O-R_4-O-\underset{\underset{O}{\|}}{C}-NH_2 \qquad (Ib)$$

worin

$R_1$ ein primäres $C_1$–$C_{18}$-Alkyl ist und

$R_4$ ein zweiwertiger Rest, der sich aus einem diprimären Dialkohol durch Entfernen der beiden Hydroxygruppen ableitet,

mit primären Dialkoholen im Molverhältnis von 1,5:1 bis 1:1,5 und mit Monoalkoholen, dadurch gekennzeichnet, dass die Umsetzung in Gegenwart von $10^{-3}$ bis 5 Mol-%, bezogen auf Mole an jeweils eingesetzten Urethanen, Katalysatoren bei Reaktionstemperaturen zwischen 120 °C und 270 °C erfolgt.

**Revendications**

1. Procédé de préparation d'esters carboniques de mono- ou poly-alcools par réaction d'uréthannes répondant aux formules Ia ou Ib

$$R_1-O-\overset{\overset{O}{\|}}{C}-NH_2 \qquad (Ia)$$

$$H_2N-\overset{\overset{O}{\|}}{C}-O-R_4-O-\underset{\underset{O}{\|}}{C}-NH_2 \qquad (Ib)$$

dans lesquelles

$R_1$ représente un groupe alkyle primaire en $C_1$–$C_{18}$ et

$R_4$ représente un reste divalent dérivant d'un dialcool diprimaire par élimination des deux groupes hydroxy,

avec des mono- ou avec des poly-alcools dans un rapport molaire alcool/groupe uréthane d'au moins 1:1, caractérisé en ce que l'on effectue la réaction en présence de $10^{-3}$ à 5 mol-%, par rapport aux moles d'uréthanne mis en œuvre dans chaque cas, de catalyseurs, en portant sous agitation à des températures d'au moins 120 °C, en portant ensuite lentement, en 5 à 10 h à 270 °C, et en laissant réagir complètement à ce niveau.

2. Procédé de préparation d'oligo- et polycarbonates par réaction de mono-uréthannes de formule Ia

$$R_1-O-\underset{\underset{O}{\|}}{C}-NH_2 \qquad (Ia)$$

ou de bis-uréthannes de formule Ib

O
||
$H_2N-C-O-R_4-O-C-NH_2$ (Ib)
||
O

dans lesquelles
$R_1$ représente un groupe alkyle primaire en $C_1-C_{18}$ et
$R_4$ représente un reste divalent dérivant d'un dialcool diprimaire par élimination des deux groupes hydroxy,
avec des dialcools primaires, dans un rapport molaire de 1,5:1 à 1:1,5 et avec des monoalcools, caractérisé en ce que la réaction est effectuée en présence de $10^{-3}$ à 5 mol-%, par rapport aux moles d'uréthanne mis en œuvre dans chaque cas, de catalyseurs à des températures de réaction de 120 à 270 °C.

**Claims**

1. Process for the preparation of carbonic acid esters of monoalcohols or polyalcohols by reaction of urethanes of the formula Ia or Ib

O
||
$R_1-O-C-NH_2$ (Ia)

O O
|| ||
$H_2N-O-R_4-O-C-NH_2$ (Ib)

wherein
$R_1$ is a primary $C_1-C_{18}$-alkyl radical and
$R_4$ is a divalent radical which is derived from a diprimary dialcohol by removal of the two hydroxyl groups,
with monoalcohols or with polyalcohols in a molar ratio of alcohol to urethane group of at least 1 to 1, characterised in that the reaction is carried out in the presence of $10^{-3}$ to 5 mol %, relative to the mols of urethanes employed in each case, of catalysts, the mixture being warmed to temperatures of at least 120 °C, with stirring, and slowly warmed to 270 °C for 5 to 10 hours and allowed to react completely.

2. Process for the preparation of oligocarbonates and of polycarbonates by reaction of monourethanes of the formula (Ia)

$R_1-O-C-NH_2$ (Ia)
||
O

or bisurethanes of the formula (Ib)

O
||
$H_2N-C-O-R_4-O-C-NH_2$ (Ib)
||
O

wherein
$R_1$ is a primary $C_1-C_{18}$-alkyl radical and
$R_4$ is a divalent radical which is derived from a diprimary dialcohol by removal of the two hydroxyl groupes,

with primary dialcohols in a molar ratio of 1.5:1 to 1:1.5 and with monoalcohols, characterised in that the reaction is carried out in the presence of $10^{-3}$ to 5 mol %, relative to the mols of urethanes employed in each case, of catalysts at reaction temperatures between 120 °C and 270 °C.